# EUROPEAN PATENT APPLICATION

(11) **EP 4 056 217 A1**
(43) Date of publication of application: **14.09.2022**
(21) Application number: 21161900.2
(22) Date of filing: 10.03.2021
(51) Int. Cl.: A61M 15/00, A61M 15/06, A61M 15/02, A61M 11/04, A24F 40/00

(54) **AEROSOL GENERATION DEVICE WITHOUT AEROSOL LEAKAGE**

(71) Applicant: JT International SA, 1202 Geneva (CH)
(72) Inventor: WRIGHT, Alec, Guildford, Surrey GU2 7SU (GB); GARCIA GARCIA, Eduardo Jose, 1218 Grand-Saconnex (CH); PILATOWICZ, Grzegorz Aleksander, 1297 Founex (CH)
(74) Representative: Serjeants LLP

(57) **Abstract**

An aerosol generation device (1) comprises a container (2-1) for storing an aerosol-forming material, an aerosol generation unit (3-1) comprising at least one micro electro-mechanical system die (12-1) arranged for transforming the aerosol-forming material into an aerosol, and a mouthpiece (4) comprising an outlet (11) through which a user inhales the aerosol. This aerosol generation unit (3-1) is installed into the mouthpiece (4) with its micro electro-mechanical system die (12-1) in the vicinity of this outlet (11) in order that the aerosol is generated in an area (13) located just before the outlet (11) to prevent deposit of aerosol on an inner face (14) of a wall of the mouthpiece (4).

## Description

### Field of the invention

The present invention relates to an aerosol generation device comprising micro electro-mechanical system die(s).

### Background

Some aerosol generation devices comprise at least one container for storing an aerosol-forming material, an aerosol generation unit comprising at least one micro electro-mechanical system (or MEMS) die arranged for transforming the aerosol-forming material into an aerosol, and a mouthpiece comprising an outlet through which a user inhales this aerosol.

When the aerosol generation device is portable, i.e. usable when held by a user, it comprises further at least one battery (or power source) possibly rechargeable and storing electrical energy that is used by each micro electro-mechanical system die for generating the aerosol. In this case the aerosol generation device may be a vaporizer or an electronic cigarette.

In the following description the term "aerosol-forming material" is used to designate any material that is aerosolizable in air to form an aerosol. It may, for instance, be in liquid form, in solid form, or in a semi liquid form. So, it may be a liquid, gel, paste or wax or the like, or any combination of these.

The aerosol-forming material may comprise one or more of nicotine, polyol, caffeine or other active components. An active component may be carried by a carrier which may include propylene glycol or glycerin, for instance. A flavoring may also be present in the aerosol-forming material and may include Ethylvanillin (vanilla), menthol, Isoamyl acetate (banana oil) or similar, for instance.

Moreover, in the following description the term "aerosol" may include a suspension of substance as one or more of solid particles, liquid droplets and gas. Such a suspension may be in a gas including air. Aerosol herein may generally refer to, or include, a vapor, and may include one or more components of the aerosol-forming material.

More, in the following description the term "micro electro-mechanical system die" (or "MEMS die") is used to designate small electronic devices comprising a plurality of small thermal firing chambers from which aerosol-forming material droplets are ejected by applying pulses of pressure to the supplied aerosol-forming material. To create these pressure pulses the thermal inkjet principle can be applied, for instance. Examples of electronic cigarettes comprising an aerosol generation unit comprising at least one MEMS die are notably described in the patent documents WO 2016/064684 and DE 102017123869.

Actually, the aerosol (generated by the MEMS die) is released into a flow path delineated by the inner face of a kind of chimney ending at the mouthpiece outlet. So, aerosol is deposited on this chimney inner face during a vaping session, and may leak from the mouthpiece outlet after this vaping session. Such a leakage may induce stains or marks in a user pocket or user bag or on a surface on which the aerosol generation device is left, which may be considered as a drawback by a lot of users.

So, the present invention aims at improving the situation.

### Summary

The proposed invention provides an embodiment of an aerosol generation device comprising at least one container for storing an aerosol-forming material, at least one aerosol generation unit comprising at least one micro electro-mechanical system die arranged for transforming the aerosol-forming material into an aerosol, and a mouthpiece comprising an outlet through which a user inhales this aerosol.

This aerosol generation device is characterized in that each aerosol generation unit is installed into the mouthpiece with its micro electro-mechanical system die in the vicinity of the mouthpiece outlet in order that the aerosol is generated in an area located just before this mouthpiece outlet to prevent deposit of aerosol on an inner face of a wall of the mouthpiece.

Thanks to the invention, when the user stops his vaping session, there is no, or significantly reduced, aerosol leakage because there is no aerosol deposited on the inner face of the mouthpiece and no more generation of aerosol.

The embodiment of aerosol generation device may comprise other features, considered separately or combined, and notably :
- in a first example of embodiment it may comprise only one aerosol generation unit comprising a printed circuit board hermetically attached to the mouthpiece inner face before the outlet and comprising each micro electro-mechanical system die facing the mouthpiece outlet;
- in this first example of embodiment the micro electro-mechanical system die may be separated from the mouthpiece outlet by a distance comprised between 3 mm and 7 mm, for instance;
- in a second example of embodiment it may comprise first and second aerosol generation units comprising respectively first and second printed circuit boards comprising respectively first and second micro electro-mechanical system dies and attached to the mouthpiece inner face before the mouthpiece outlet in such a way that the first and second micro electro-mechanical system dies are facing each other to generate the aerosol therebetween;
- in this second example of embodiment it may comprise first and second conduits coupled to each container and respectively to the first and second printed circuit boards for feeding the first and second micro electro-mechanical system dies with aerosol-forming material;
- it may comprise a filter coupled to each container and to each micro electro-mechanical system die and arranged for filtering the aerosol-forming material before the latter reaches each micro electro-mechanical system die;
- the filter may comprise two outlets connected respectively to the first and second conduits for feeding them separately with filtered aerosol-forming material;
- the filter may be installed into the mouthpiece;
- it may comprise two containers comprising a same aerosol-forming material, or two containers comprising two different aerosol-forming materials;
- it may comprise a battery storing electrical energy that is used by each micro electro-mechanical system die for generating the aerosol;
- the battery may be rechargeable;
- it may constitute an electronic cigarette.

### Brief description of the figure

The invention and its advantages will be better understood upon reading the following detailed description, which is given solely by way of non-limiting examples and which is made with reference to the appended drawings, in which:
- the figure 1 (FIG.1) schematically and functionally illustrates a first example of embodiment of an aerosol generation device according to the invention, and
- the figure 2 (FIG.2) schematically and functionally illustrates a second example of embodiment of an aerosol generation device according to the invention.

### Detailed description of embodiments

The invention aims at offering an aerosol generation device 1 which is subject to a significantly reduced leakage, and if possible not subject at all to aerosol leakage, after a vaping session.

In the following description it will be considered that the aerosol generation device 1 is (or constitutes) an electronic cigarette (or e-cigarette or else personal vaporizer). But an aerosol generation device according to the invention could be of another type, as soon as it allows to transform an aerosol-forming material into an aerosol. It is in particular usable with an aerosol producing technology that produces a (close to) room temperature aerosol.

It is recalled that an "aerosol-forming material" is used to designate any material that is aerosolizable in air to form an aerosol. It may, for instance, be in liquid form, in solid form, or in a semi liquid form. So, it may be a liquid, gel, paste or wax or the like, or any combination of these, and may comprise one or more of nicotine, polyol, caffeine or other active components, or else flavoring.

In the following description it will be considered that the aerosol-forming material(s) is/are in a liquid form (or state). But if the aerosol-forming material(s) is/are not already provided by a container (or tank) 2-j as a liquid, the transformation of an aerosol-forming material into a liquid state can be obtained by heating.

It is also recalled that the term "aerosol" may include a suspension of substance as one or more of solid particles, liquid droplets and gas, and that such a suspension may be in a gas including air.

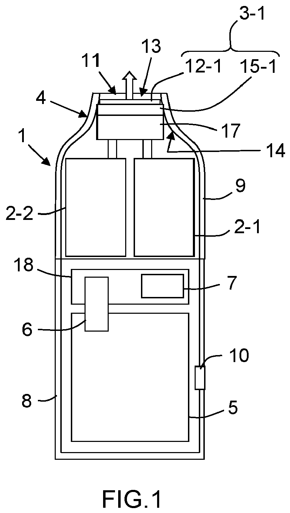

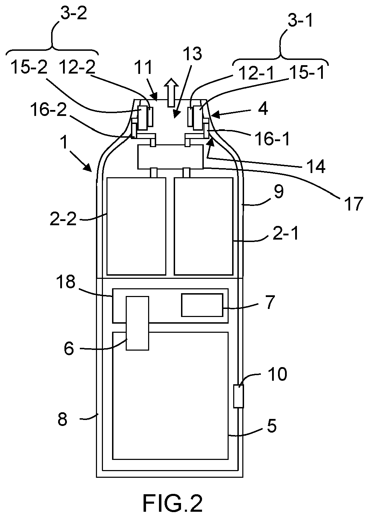

As illustrated in figures 1 and 2 an aerosol generation device 1 according to the invention comprises at least one container (or tank) 2-j, at least one aerosol generation unit 3-k, a mouthpiece 4, and also preferably a battery (or power source) 5 and a user interface 6. It further comprises a control device 7 arranged for controlling at least the working of each aerosol generation unit 3-k.

For instance, and as illustrated in the non-limiting examples of figures 1 and 2, the control device 7 and the possible battery 5 and user interface 6 may belong to a first body 8 to which a second body 9 is coupled (for instance by magnets, clipping, or screwing by means of two corresponding threaded portions, as non-limiting examples). Also for instance, and as illustrated in the non-limiting examples of figures 1 and 2, the control device 7 may be fixed onto a printed circuit board 18 (here housed into the first body 8). The possible user interface 6 may be also fixed onto this printed circuit board 18, as illustrated. Also for instance, and as illustrated in the non-limiting examples of figures 1 and 2, the container(s) 2-j, aerosol generation unit(s) 3-k and mouthpiece 4 may belong to the second body 9. But in a variant the aerosol generation device 1 could comprise a single body comprising all its elements (or components).

The battery 5 is arranged for storing electrical energy that is necessary to the working of the aerosol generation unit(s) 3-k, control device 1 and user interface 6. For instance, the battery 5 may be rechargeable, and in this case, and as illustrated in the non-limiting examples of figures 1 and 2, the first body 8 may comprise an electrical connector 10 to which a charger cable may be connected during a charging session of the battery 5. Such a charger cable may be coupled to an (AC) adapter or to a wall socket. The charger cable and/or the (AC) adapter may belong to the aerosol generation device 1.

The possible user interface 6 is coupled to the control device 7, at least arranged for allowing the user to control the control device 7 and at least for switching on and switching off the aerosol generation device 1. So, it may comprise a button and/or a digital display (or screen) arranged for displaying information relative to a current vaping session or a possible current charging session.

Each container 2-j is arranged for storing an aerosol-forming material. In the non-limiting examples illustrated in figures 1 and 2 the aerosol generation device 1 comprises first 2-1 (j = 1) and second 2-2 (j = 2) containers. But in a variant not illustrated it may comprise only one container 2-j.

In the case where the aerosol generation device 1 comprises first 2-1 and second 2-2 containers, they may both store the same aerosol-forming material, or they may store two different aerosol-forming materials (for instance one may comprise nicotine while the other one may comprise a flavor).

The mouthpiece 4 comprises an outlet 11 through which the user of the aerosol generation device 1 inhales the aerosol generated by each aerosol generation unit 3-k. This mouthpiece 4 may be integral with the possible second body 9 (as illustrated) or may be an interchangeable part of the possible second body 9.

Each aerosol generation unit 3-k comprises at least one micro electro-mechanical system die 12-k arranged for transforming the aerosol-forming material (initially stored in a container 2-j) into an aerosol. In the non-limiting first example illustrated in figure 1 the aerosol generation device 1 comprises only one aerosol generation unit 3-1 (k = 1), while in the non-limiting second example illustrated in figure 2 the aerosol generation device 1 comprises first 3-1 (k = 1) and second 3-2 (k = 2) aerosol generation units.

As illustrated, each aerosol generation unit 3-k is installed into the mouthpiece 4 with its micro electro-mechanical system die 12-k in the vicinity of the mouthpiece outlet 11 in order that the aerosol is generated in an area 13 located just before this mouthpiece outlet 7 to prevent deposit of aerosol on an inner face 14 of a wall of the mouthpiece 4. Here, the term "vicinity" means very near from the mouthpiece outlet 11 (typically some millimeters), and therefore the term "just before" means still nearer from the mouthpiece outlet 12 than a micro electro-mechanical system die 12-k.

The aerosol being now generated just before the mouthpiece outlet 11, when the user stops his vaping session, there is no aerosol leakage, or at least a significantly reduced leakage, because there is no aerosol deposited on the inner face 11 of the mouthpiece 4 and no more generation of aerosol. This aerosol generation just before the mouthpiece outlet 11 is not dangerous because the aerosol is generated at the ambient temperature and therefore doesn't need to be cooled before reaching the mouth of the user.

For instance, each micro electro-mechanical system die 12-k may comprise small chambers each containing a heater therein. Each heater is arranged for heating the aerosol-forming material until the water it contains vaporizes and a gas bubble is created. The gas bubble is comprised of a phase change of the aerosol-forming material, usually liquid, and potentially air trapped in the liquid. The amount of the aerosol-forming material being boiled is about 1% of the total amount. In other words, around 1% of the aerosol-forming material is superheated to form a gas bubble. This 1% consists of the amount of aerosol-forming material that is the closest to the heater. Gas being much more voluminous than liquid, it provides the force to push out from the aerosol generation unit 3-k. This allows approximately 80-90% of the aerosol-forming material above the gas bubble to be ejected. Gas bubbles grow as they are heated until being large enough that they force liquid droplets to be ejected. The gas bubbles also escape when the liquid droplets are ejected. This creates a vacuum which causes more liquid to be drawn into the aerosol generation unit 3-k from a container 2-j. The process then repeats.

It shall be noted that the propylene glycol (PG) and the vegetable glycerine (VG) that may be present in the aerosol-forming material may not vaporize as boiling points of these components are higher than the boiling point of water at the same atmospheric pressure. However, because of the high temperature of the heater, it is very possible that all of the aerosol-forming material near the heater, regardless of composition, is superheated and undergoes the phase change to the gas bubble. In other words, the 1% amount of the aerosol-forming material that is superheated can be made up of a mixture of components that is similar to that of the rest of the aerosol-forming material.

In the non-limiting first example illustrated in figure 1, where the aerosol generation device 1 comprises only one aerosol generation unit 3-1, the latter (3-1) may comprise a printed circuit board 15-1 hermetically attached to the inner face 14 of the mouthpiece 4 before (or upstream of) the mouthpiece outlet 11. This printed circuit board 15-1 comprises the micro electro-mechanical system die(s) 12-k of its aerosol generation unit 3-1 in a position facing the mouthpiece outlet 11. In this first example the printed circuit board 15-1 is preferably parallel to the mouthpiece outlet 11, but this is not mandatory.

For instance, and as non-limiting example, the micro electro-mechanical system die(s) 12-1 may be separated from the mouthpiece outlet 11 by a distance comprised between 3 mm and 7 mm. In this case, this distance may be equal to 5 mm, for instance.

The printed circuit board 15-1 being hermetically attached to the inner face 14 of the mouthpiece 4, it comprises at least one through-hole allowing the air, coming from outside (through at least one inlet defined in the first 8 or second 9 body) and sucked by the user during an inhalation phase (or "puff"), to cross it to reach the area 13 where it is mixed with the generated aerosol. Alternative implementations are also possible.

In the non-limiting second example illustrated in figure 2, where the aerosol generation device 1 comprises first 3-1 and second 3-2 aerosol generation units, the latter (3-k) may comprise respectively first 15-1 and second 15-2 printed circuit boards comprising respectively first 12-1 and second 12-2 micro electro-mechanical system dies. These first 15-1 and second 15-2 printed circuit boards are attached to the inner face 14 of the mouthpiece 4 before (or upstream) the mouthpiece outlet 11 in such a way that the first 12-1 and second 12-2 micro electro-mechanical system dies are facing each other to generate the aerosol therebetween in the area 13. In this second example the printed circuit boards 15-k are preferably perpendicular to the mouthpiece outlet 11 and therefore parallel one to the other, but this is not mandatory.

For instance, and as non-limiting example, the part of each micro electro-mechanical system die(s) 12-1 that is the nearest from the mouthpiece outlet 11 may be separated from the latter (11) by a distance comprised between 2 mm and 6 mm. In this case, this distance may be equal to 4 mm, for instance. A short distance gives a more compact aerosol generation device 1, and less risk of depositing aerosol inside the aerosol generation device 1.

Due to the small drop size, the inertia within each droplet is very small. So, the direction of the aerosol is easily manipulated by the airflow. Therefore, as the user inhales and the micro electro-mechanical system dies 12-k are fired, the aerosol is initially fired to the opposite printed circuit board 15-k before the user's puff pulls the aerosol from the mouthpiece outlet 11. This configuration leaves a void (or area 13) at the top of the mouthpiece 4 between the aerosol generation units 3-k. When the aerosol generation device 1 is not in use, this void could be filled with a plug cap by the user. Such a plug cap could have, for instance, foam elements which could wipe the top of the micro electro-mechanical system dies 12-k when inserted and removed to keep them clean and also capture a possible excess of liquid. This plug cap would also prevent any dirt from entering the mouthpiece 4 through its outlet 11 when the aerosol generation device 1 is in the user's pocket, for instance.

As illustrated in figure 2, in the second example the aerosol generation device 1 may also comprise first 16-1 and second 16-2 conduits coupled to each container 2-j and respectively to the first 15-1 and second 15-2 printed circuit boards for feeding the first 12-1 and second 12-2 micro electro-mechanical system dies with aerosol-forming material. So, the first 15-1 and second 15-2 printed circuit boards comprises respectively through-holes allowing coupling between the first 16-1 and second 16-2 conduits and the inlets of the first 12-1 and second 12-2 micro electro-mechanical system dies.

For instance, and as illustrated in the non-limiting examples of figures 1 and 2, the aerosol generation device 1 may also comprise a filter 17 coupled to each container 2-j and to each micro electro-mechanical system die 12-k (possibly via the first 16-1 and second 16-2 conduits). This filter 17 is arranged for filtering the aerosol-forming material coming from each container 2-j before it reaches each micro electro-mechanical system die 12-k. This filter 17 prevents the passage of particles from each container 2-j to the aerosol generation unit(s) 3-k. Such particles, which could be introduced into a container 2-j during its filling and/or which could come from a poor quality aerosol-forming material, could interfere with the operation of an aerosol generation unit 3-k. In particular, they could plug the small thermal firing chambers of the micro electro-mechanical system die(s) 12-k.

For instance, this filter 17 may comprise a chamber comprising a filtering means that is crossed by the aerosol-forming material coming from each container 2-j. This filtering means may be a mesh (for instance a metallic mesh, and preferably a stainless-steel mesh).

Also for instance, and as illustrated in the non-limiting second example of figure 2, the filter 17 may comprise two outlets connected respectively to the first 16-1 and second 16-2 conduits for feeding them separately with filtered aerosol-forming material. In the case where the aerosol generation device 1 comprises first 2-1 and second 2-2 containers, as illustrated in figures 1 and 2, the filter 17 comprises two inlets coupled respectively to these first 2-1 and second 2-2 containers. So, the aerosol-forming materials coming from these first 2-1 and second 2-2 containers are mixed inside the filter 17 before reaching the aerosol generation unit(s) 3-k.

Also for instance, and as illustrated in the non-limiting examples of figures 1 and 2, the filter 17 may be installed into the mouthpiece 4. But this is not mandatory. Indeed it may be located into the second body 9 between the container(s) 2-j and the mouthpiece 4 (comprising the aerosol generation unit(s) 3-k).

Also for instance, to prevent any ingress of the user's tongue into the mouthpiece outlet 11 and therefore any contact with an aerosol generation unit 3-k, which could damage its micro electro-mechanical system die(s) 12-k, a cross bar may be added across the mouthpiece outlet 11. But it is also possible to use a very narrow mouthpiece outlet 11.

It should be appreciated by those skilled in the art that some block diagrams of figures 1 and 2 herein represent conceptual views of illustrative circuitry embodying the principles of the invention.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

## Claims

1. Aerosol generation device (1) comprising at least one container (2-j) for storing an aerosol-forming material, at least one aerosol generation unit (3-k) comprising at least one micro electro-mechanical system die (12-k) arranged for transforming said aerosol-forming material into an aerosol, and a mouthpiece (4) comprising an outlet (11) through which a user inhales said aerosol, wherein each aerosol generation unit (3-k) is installed into said mouthpiece (4) with its micro electro-mechanical system die (12-k) in the vicinity of said outlet (11) in order that said aerosol is generated in an area (13) located just before said outlet (11) to prevent deposit of aerosol on an inner face (14) of a wall of said mouthpiece (4).

2. Aerosol generation device according to claim 1, wherein it comprises one aerosol generation unit (3-1) comprising a printed circuit board (15-1) hermetically attached to said inner face (14) before said outlet (11) and comprising said micro electro-mechanical system die (12-k) facing said outlet (11).

3. Aerosol generation device according to claim 2, wherein said micro electro-mechanical system die (12-k) is separated from said outlet (11) by a distance comprised between 3 mm and 7 mm.

4. Aerosol generation device according to claim 1, wherein it comprises first (3-1) and second (3-2) aerosol generation units comprising respectively first (15-1) and second (15-2) printed circuit boards comprising respectively first (12-1) and second (12-2) micro electro-mechanical system dies and attached to said inner face (14) before said outlet (11) in such a way that said first (12-1) and second (12-2) micro electro-mechanical system dies are facing each other to generate said aerosol therebetween.

5. Aerosol generation device according to claim 4, wherein it comprises first (16-1) and second (16-2) conduits coupled to each container (2-j) and respectively to said first (15-1) and second (15-2) printed circuit boards for feeding said first (12-1) and second (12-2) micro electro-mechanical system dies with aerosol-forming material.

6. Aerosol generation device according to one of claims 1 to 5, wherein it comprises a filter (17) coupled to each container (2-j) and to each micro electro-mechanical system die (12-k) and arranged for filtering said aerosol-forming material before the latter reaches each micro electro-mechanical system die (12-k).

7. Aerosol generation device according to the combination of claims 5 and 6, wherein said filter (17) comprises two outlets connected respectively to said first (16-1) and second (16-2) conduits for feeding them separately with filtered aerosol-forming material.

8. Aerosol generation device according to claim 6 or 7, wherein said filter (17) is installed into said mouthpiece (4).

9. Aerosol generation device according to one of claims 1 to 8, wherein it comprises two containers (2-j) comprising a same aerosol-forming material.

10. Aerosol generation device according to one of claims 1 to 8, wherein it comprises two containers (2-j) comprising two different aerosol-forming materials.

11. Aerosol generation device according to one of claims 1 to 10, wherein it comprises a battery (5) storing electrical energy that is used by each micro electro-mechanical system die (12-k) for generating said aerosol.

12. Aerosol generation device according to claim 11, wherein said battery (5) is rechargeable.

13. Aerosol generation device according to one of the preceding claims, wherein it constitutes an electronic cigarette.
